# NOUVEAU FASCICULE DE BREVET EUROPEEN

(11) **EP 0 705 592 B2**
(45) Date de publication et mention de la décision concernant l'opposition: **14.06.2006**
(45) Mention de la délivrance du brevet: 27.11.1996
(21) Numéro de dépôt: 95401084.9
(22) Date de dépôt: 10.05.1995
(51) Int. Cl.: A61K 8/06, A61K 8/60, A61K 8/92, A61Q 1/14, A61Q 19/10

(54) **Emulsion nettoyante huile-dans-eau ayant l'aspect d'un lait**
Wie Milch aussehende Öl-in-Wasser reinigende Emulsion
Oil in water cleaning emulsion with an appearance of milk

(30) Priorité: 14.06.1994 FR 9407252
(43) Date de publication de la demande: 10.04.1996
(73) Titulaire: L'OREAL, 75008 Paris (FR)
(72) Inventeur: Marion, Catherine, F-92330 Sceaux (FR); Louvet, Nathalie, F-94550 Chevilly Larue (FR); Lukassen, Liliane, F-94550 Chevilly Larue (FR)
(74) Mandataire: Dossmann, Gérard

(56) Documents cités:
- EP-A- 0 422 862
- EP-A- 0 603 078
- EP-A- 0 629 396
- WO-A-92/06778

## Description

La présente invention se rapporte à une émulsion nettoyante huile-dans-eau (H/E) ayant l'aspect d'un lait, utilisable pour nettoyer la peau du visage et/ou du corps, éventuellement avant un traitement cosmétique et/ou dermatologique. En particulier, cette émulsion peut consister en un lait démaquillant.

L'invention a également pour objet l'utilisation de cette émulsion pour nettoyer la peau en profondeur et un procédé pour nettoyer la peau.

On sait que les émulsions huile-dans-eau et eau-dans-huile utilisées dans les domaines cosmétique et dermatologique comprennent généralement, pour faciliter leur émulsification, des émulsionnants, notamment des émulsionnants non-ioniques, dont la fonction est de rendre compatibles les phases grasse et aqueuse.

Ces émulsions, suivant leur consistance, sont appelées "laits" pour les préparations les plus fluides et "crèmes" pour les émulsions plus fermes. Un produit nettoyant se présente généralement sous forme d'un lait plus ou moins fluide. L'intérêt d'un lait est de s'étaler plus facilement, d'enrober plus facilement les salissures du fait de sa fluidité et donc de pouvoir nettoyer la peau plus en profondeur qu'une crème. Un lait doit avoir une texture assez fluide pour pouvoir s'écouler d'un flacon et bien s'étaler sur la peau. Habituellement, un lait a une viscosité égale ou inférieure à 1 Pa.s.

Les émulsionnants généralement utilisés pour la préparation des émulsions comportent des molécules d'oxyde d'éthylène. Ces émulsionnants présentent l'inconvénient d'être plus ou moins irritants.

En vue d'éviter l'emploi de ces émulsionnants irritants, il a été proposé dans le document FR-A-2668080 l'utilisation d'une composition auto-émulsionnable à base d'alcools gras.

Toutefois, les émulsions obtenues par l'utilisation d'une telle composition ne permettent pas d'obtenir une texture à la fois suffisamment fluide et stable pour avoir un lait. On peut obtenir une texture plus fluide en abaissant le taux d'émulsionnant, mais dans le cas de la composition auto-émulsionnable à base d'alcools gras, on aboutit alors à une composition qui n'est pas stable.

De plus, si l'on veut obtenir un lait démaquillant, l'introduction d'une huile démaquillante dans une émulsion peut la déstabiliser, et ceci est notamment le cas dans la composition auto-émulsionnable à base d'alcool gras telle que décrite dans le document FR-A-2668080 ; il en résulte une formulation dont la stabilité n'est pas satisfaisante.

Il subsiste donc le besoin d'une émulsion stable contenant un émulsionnant non irritant, et ayant la texture d'un lait.

L'émulsion selon l'invention permet notamment de résoudre les problèmes mentionnés ci-dessus. En effet, la demanderesse a trouvé de manière surprenante qu'il était possible d'obtenir un lait stable en utilisant comme émulsionnant une composition auto-émulsionnable à base d'alcools gras et comme gélifiant un copolymère acrylique.

La présente invention a donc pour objet une émulsion nettoyante huile-dans-eau ayant l'aspect d'un lait, caractérisée en ce qu'elle a une viscosité égale ou inférieure à 1 Pa.s et en ce qu'elle comprend comme émulsionnant une composition auto-émulsionnable à base d'alcools gras et comme gélifiant, un copolymère acrylique lequel est un copolymère réticulé d'un acide monoéthylénique en C₃-C₆ ou de son anhydride avec un ester acrylique.

L'invention a encore pour objet une utilisation de l'émulsion définie ci-dessus pour nettoyer la peau du visage et/ou du corps.

L'invention a encore pour objet un procédé pour nettoyer la peau consistant à appliquer l'émulsion définie ci-dessus sur la peau.

L'invention a enfin pour objet un lait démaquillant caractérisé en ce qu'il contient l'émulsion définie ci-dessus et une huile démaquillante.

Le choix particulier d'un copolymère acrylique comme gélifiant permet d'obtenir une émulsion suffisamment fluide pour constituer un lait tout en ayant une bonne stabilité, alors que d'autres gélifiants, tels que par exemple les polyacrylamides comme le produit commercialisé sous la dénomination de Sepigel 305 par la société Seppic, ne permettent pas d'atteindre ce résultat. Or, rien ne permettait de conduire l'homme de métier à choisir plus particulièrement ce type de gélifiant plutôt qu'un autre.

L'émulsion obtenue selon l'invention a la propriété d'être non seulement non irritante et très stable, mais encore d'être très agréable à utiliser, et d'avoir en particulier une très grande douceur à l'application. De plus, quand elle contient une huile démaquillante, elle constitue un lait démaquillant particulièrement efficace, supérieur aux laits classiquement utilisés. Ce lait démaquillant peut être utilisé aussi bien pour nettoyer le visage que les yeux.

Le copolymère acrylique utilisable dans la présente invention est en particulier un copolymère réticulé d'un acide monoéthylénique en C₃ - C₆ ou de son anhydride avec un ester acrylique à longue chaîne. L'agent réticulant est de préférence un éther allylique de glucide ou de polyol tel que le pentaérythritol ou avec un dérivé de glycol tel que le divinylglycol.

La proportion d'acide monoéthylènique présent dans le copolymère peut aller de 50 à 99 % en poids, et mieux de 90 à 98 % en poids, et la proportion d'ester acrylique peut aller de 50 à 1 % en poids, et mieux de 10 à 2 % en poids.

L'acide monoéthylènique peut être en particulier un acide acrylique de formule: 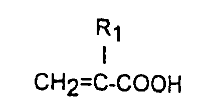 où R₁ représente un groupement choisi parmi l'hydrogène, ou les radicaux halogène, hydroxyle, lactone ou lactame, cyanogène, alkyle, aryle, aralkyle, alkylaryle ou cycloaliphatique.

Les acides monoéthylèniques de préférence utilisés pour la préparation du copolymère sont les acides acrylique et maléique et leurs dérivés.

L'ester acrylique utilisable pour la préparation du copolymère a de préférence pour formule : 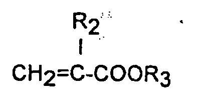 où R₂ représente un groupement choisi parmi l'hydrogène ou les radicaux méthyle ou éthyle, et R₃ représente un groupement choisi parmi les radicaux alkyle en C₈-C₃₀, oxyalkylène en C₈-C₃₀ ou carbonyloxyalkylène en C₈-C₃₀. Les radicaux alkyle en C₁₀-C₂₂ sont préférés.

Parmi les esters que l'on préfère, on peut citer les acrylates et méthacrylates de décyle, de lauryle, de stéaryle, de béhényle et de mélissyle.

Le copolymère utilisé dans la présente invention est de préférence un copolymère d'acrylate d'alkyle en C₁₀-C₃₀ et d'acide acrylique ou méthacrylique, réticulé avec un éther allylique de sucrose ou de pentaérythritol, et il est notamment choisi parmi ceux commercialisés sous les dénominations Pemulen TR1, Pemulen TR2 et Carbopol 1342 par la société Goodrich.

Ledit copolymère peut être présent à une teneur allant de 0.02 à 0,5 % en poids par rapport au poids total de l'émulsion, et de préférence de 0,05 à 0,3 % en poids.

La composition auto-émulsionnable utilisée dans la présente invention comme émulsionnant comprend avantageusement de 60 à 90 % en poids d'au moins un alcool gras ayant de 12 à 22 atomes de carbone, de 10 à 40 % en poids d'un alkylpolyoside dont la chaîne alkyle a de préférence de 12 à 22 atomes de carbone, et 0 à 5 % en poids, et par exemple de 0,5 à 5 % en poids, de polyoside. Selon un mode de réalisation particulier de l'invention, le radical alkyle de l'alkylpolyoside est identique à celui de l'alcool gras de la composition auto-émulsionnable.

Parmi les compositions auto-émulsionnables utilisables dans l'invention, on peut citer celles décrites dans la demande de brevet FR-A-2668080, et notamment le produit commercialisé sous la dénomination de Montanov 68 par la société Seppic.

Cette composition auto-émulsionnable peut être présente à une teneur allant de 0,5 à 5% en poids par rapport au poids total de l'émulsion, et de préférence allant de 1 et 3,5 % en poids par rapport au poids total de l'émulsion.

Les émulsions de l'invention trouvent en particulier leur application pour le nettoyage de la peau du corps, du cou et du visage, y compris pour le nettoyage des enfants en bas âge, et pour le démaquillage.

La phase huileuse de l'émulsion selon l'invention comprend au moins une huile animale, une huile végétale, une huile minérale, une huile siliconée, une huile fluorée et/ou une huile synthétique. La phase huileuse de l'émulsion peut contenir aussi des alcools gras ou des acides gras, et également des détergents tels que par exemple les copolymères blocs oxyéthylénés oxyropylénés appelés poloxamers.

Lorsque l'émulsion selon l'invention constitue un lait démaquillant, la phase huileuse contient aussi une huile démaquillante. On peut notamment citer comme huiles démaquillantes les esters d'acides gras, et notamment les esters ayant un nombre total d'atomes de carbone choisi entre 12 et 20, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.

L'ester utilisé comme huile démaquillante peut être en particulier choisi dans le groupe constitué par l'adipate de dioctyle, le palmitate d'éthyl-2 hexyle, l'adipate de di-isopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprytate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.

L'émulsion selon l'invention peut contenir de 2 à 30 % en poids d'huile, et de préférence de 5 à 25 % en poids par rapport au poids total de l'émulsion.

De façon connue, l'émulsion de l'invention peut, en outre, contenir des adjuvants couramment utilisés dans les domaines cosmétique et/ou dermatologique, tels que les conservateurs, les antioxydants, les parfums, les charges, les filtres, les séquestrants, les huiles essentielles, les matières colorantes, les actifs hydrophiles ou lipophiles, et encore des vésicules lipidiques. Ces adjuvants sont utilisés, selon leur nature, dans les proportions habituelles des compositions cosmétiques et/ou dermatologiques, par exemple de 0,01 à 10 % en poids par rapport au poids total de l'émulsion, et mieux de 0,1 à 6 % en poids.

Les actifs sont par exemple choisis parmi les hydratants tels que, notamment, les polyols comme la glycérine, et les hydrolysats de protéine ; les anti-inflammatoires ; les agents anti-radicaux libres comme le tocophérol (vitamine E) ou ses dérivés ; les dépigmentants : les actifs biologiques tels que l'urée, les acides aminés, les vitamines et leurs dérivés, les protéines, l'acide salicylique et ses dérivés, les α-hydroxy-acides, l'acide pyrrolidone carboxytique et ses sels, les céramides, les extraits végétaux (extrait de racine de guimauve).

Les émulsions huile-dans-eau selon l'invention sont obtenues selon les méthodes classiques par mélange à chaud, sous forte agitation, à une température d'environ 70°C, de la phase huileuse contenant l'émulsionnant et les adjuvants lipophiles, à la phase aqueuse comprenant le gélifiant et les adjuvants hydrophiles, puis refroidissement jusqu'à la température ambiante.

La description qui suit est en référence avec les figures 1 à 3 annexées qui sont des photographies à l'échelle x 250 d'une émulsion selon l'invention et d'émulsions données à titre de comparaison.

Les exemples suivants illustrent l'invention. Dans ces exemples, les proportions indiquées sont des pourcentages en poids.

### Exemple 1 : Lait démaquillant

| | |
|---|---|
| Palmitate d'éthyle-2 hexyle | 20,0 |
| Hydroxyde de sodium (neutralisant) | 0,03 |
| Copolymère d'acrylates/C₁₀-C₃₀-alkylacrylate (Pemulen TR1 de la société Goodrich) | 0,1 |
| Cétéaryl glucoside (Montanov 68 de la société Seppic) | 2,45 |
| Glycérine | 5,0 |
| Conservateurs | 0,35 |
| Eau | qsp 100 % |

L'émulsion obtenue est un lait de viscosité de 0,8 Pa.s. Ce lait est très doux à l'application et est très efficace pour le démaquillage du visage.

La figure 1 est une photographie à l'échelle x 250 de l'émulsion obtenue. On constate sur cette photographie que les globules d'huile sont homogènes et bien répartis dans la phase aqueuse.

Le pouvoir démaquillant de ce lait a été testé sur un panel de 49 femmes. 98 % de ces femmes l'ont jugé excellent. 90 % de ces utilisatrices ont trouvé d'autre part qu'après utilisation de ce lait, la peau restait nette, propre et non grasse. Enfin, 80 % de ces mêmes femmes ont trouvé ce lait très doux.

### Exemple comparatif 1 (exemple 3 du document FR-A-2668080)

| | |
|---|---|
| Cétéaryl glucoside (Montanov 68) | 5,0 |
| Huile d'amandes douces | 5,0 |
| Polyacrylamide (Sepigel 305) (Gélifiant) | 0,3 |
| Glycérine | 5,0 |
| Conservateur | 0,2 |
| Eau | qsp 100 % |

L'émulsion obtenue constitue une crème épaisse et non un fait Elle présente une viscosité de 1,36 Pa.s et ne s'écoule pas du récipient la contenant. En outre, cette émulsion ne démaquille nullement. Elle se fluidifie moins, mouille moins la peau et assure donc un nettoyage moins efficace.

La figure 2 est une photographie à l'échelle x 250 de l'émulsion obtenue. L'huile est moins bien dispersée que dans l'exemple 1 de l'invention.

### Exemple comparatif 2

Dans cet exemple, on a remplacé l'huile d'amandes douces de l'exemple précédent par une huile démaquillante : le palmitate d'éthyle-2 hexyle. La composition est donc la suivante :

| | |
|---|---|
| Cétéaryl glucoside | 5,0 |
| Palmitate d'éthyle-2 hexyle | 20,0 |
| Polyacrylamide (Sepigel 305) (Gélifiant) | 0,3 |
| Glycérine | 5,0 |
| Conservateur | 0,2 |
| Eau | qsp 100 % |

L'émulsion obtenue constitue une crème épaisse et non un lait. Elle présente une viscosité de 1,86 Pa.s et elle ne s'écoule pas du récipient la contenant. On constate donc, par rapport à l'exemple comparatif 1, que l'introduction de l'huile démaquillante a entraîné une augmentation de la viscosité. De plus, malgré la présence de l'huile démaquillante, la composition obtenue démaquille très médiocrement

Enfin, cette émulsion a un aspect très grossier et elle est peu stable, et ceci apparaît clairement sur la figure 3 qui est une photographie de l'émulsion à la même échelle que les photographies précédentes. Ici, les globules d'huile ont une taille de 13 mm au lieu de 2 mm.

### Exemple 2 : lait nettoyant pour bébé

| | |
|---|---|
| Huile d'amandes douces | 7,00 |
| Huile de vaseline | 13,00 |
| Copolymère d'acrytales/C₁₀-C₃₀-alkylacrylate (Pemulen TR1 de la société Goodrich) | 0,1 |
| Hydroxyde de sodium (neutralisant) | 0,03 |
| Cétéaryl glucoside (Montanov 68 de la société Seppic) | 2,45 |
| Glycérine | 3.00 |
| Eau de rose | 3.00 |
| Conservateurs | 0,35 |
| Eau | qsp 100 % |

L'émulsion obtenue constitue un lait nettoyant particulièrement doux à utiliser.

### Exemple 3 : lait adoucissant pour le corps

| | |
|---|---|
| Huile de rosier muscat | 5,00 |
| Huile de carthame | 3,00 |
| Huile de vaseline | 14,00 |
| Copolymère d'acrylates/C₁₀-C₃₀-alkylacrylate (Pemulen TR1 de la société Goodrich) | 0,10 |
| Hydroxyde de sodium (neutralisant) | 0,03 |
| Cétéaryl glucoside (Montanov 68 de la société Seppic) | 2,45 |
| Glycérine | 5.00 |
| Extrait de racine de guimauve | 3,00 |
| Eau | qsp 100% |

L'émulsion obtenue constitue un lait qui laisse une peau très douce.

### Exemple 3 : lait de toilette pour le visage et les yeux

| | |
|---|---|
| Copolymère d'acrylates/C₁₀-C₃₀-alkylacrylate (Pemulen TR2 de la société Goodrich) | 0,10 |
| Hydroxyde de sodium (neutralisant) | 0,03 |
| Cétéaryl glucoside (Montanov 68 de la société Seppic) | 2,00 |
| Adipate de dioctyle | 15,00 |
| Poloxamer 184 | 0,5 |
| Conservateur | 0,1 |
| Eau | qsp 100 % |

L'émulsion obtenue constitue un lait qui permet un nettoyage efficace du visage et des yeux et qui est très doux.

## Revendications

1. Emulsion nettoyante huile-dans-eau ayant l'aspect d'un lait, **caractérisée en ce qu'**elle a une viscosité égale ou inférieure à 1 Pa.s et **en ce qu'**elle comprend comme émulsionnant une composition auto-émulsionnable à base d'alcools gras et comme gélifiant un copolymère acrylique lequel est un copolymère réticulé d'un acide monoéthylénique en C₃-C₆ ou de son anhydride avec un ester acrylique.

2. Emulsion nettoyante selon la revendication 1, **caractérisée en ce que** l'acide monoéthylènique a pour formule : 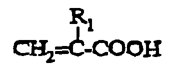 où R₁ représente un groupement choisi parmi l'hydrogène ou les radicaux halogène, hydroxyle, lactone, lactame, cyanogène, alkyle, aryle, aralkyle, alkyaryle et cycloaliphatique.

3. Emulsion nettoyante selon la revendication 1 ou 2, **caractérisée en ce que** l'ester acrylique a pour formule : 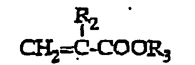 où R₂ représente un groupement choisi parmi l'hydrogène ou les radicaux méthyle ou éthyle, et R₃ représente un groupement choisi parmi les radicaux alkyle en C₈-C₃₀, oxyalkylène en C₈-C₃₀ et carbonyloxyalkylene en C₈-C₃₀.

4. Emulsion nettoyante selon l'une quelconque des revendications 1 à 3 **caractérisée en ce que**, dans le copolymère, la proportion d'acide monoéthylènique est de 50 à 99 % en poids et la proportion d'ester acrylique est de 50 à 1 % en poids.

5. Emulsion nettoyante selon l'une quelconque des revendications 1 à 4, **caractérisée en ce que** le copolymère est un copolymère d'acrylate d'alkyle en C₁₀-C₃₀ et d'acide acrylique ou méthacrylique, réticulé avec un éther allylique de sucrose ou de pentaérythritol.

6. Emulsion nettoyante selon l'une quelconque des revendications précédentes, **caractérisée en ce que** la composition auto-émulsionnable comprend de 60 à 90 % en poids d'au moins un alcool gras ayant de 12 à 22 atomes de carbone, de 10 à 40 % en poids d'un alkylpolyoside dont la chaîne alkyle a de 12 à 22 atomes de carbone, et de 0 à 5 % en poids de polyoside.

7. Emulsion nettoyante selon l'une quelconque des revendications précédentes, **caractérisée en ce qu'**elle comprend de 0.5 à 5% en poids de composition auto-émulsionnable par rapport au poids total de l'émulsion.

8. Emulsion nettoyante selon l'une quelconque des revendications précédentes, **caractérisée en ce qu'**elle comprend de 0,02 à 0,5 % en poids de copolymère par rapport au poids total de l'emulsion.

9. Emulsion nettoyante selon l'une quelconque des revendications précédentes, **caractérisée en ce qu'**elle comprend de 2 à 30 % en poids d'huile.

10. Emulsion nettoyante selon l'une quelconque des revendications précédentes, **caractérisée en ce qu'**elle comprend en outre au moins un adjuvant choisi parmi les conservateurs, les antioxydants, les parfums, les charges, les filtres, les séquestrants, les huiles essentielles, les matières colorantes, les actifs hydrophiles ou lipophiles, et les vésicules lipidiques.

11. Lait démaquillant **caractérisé en ce qu'**il contient une émulsion selon l'une quelconque des revendications 1 à 10 et une huile démaquillante.

12. Utilisation de l'émulsion nettoyants selon l'une quelconque des revendications 1 à 10 pour nettoyer la peau.

13. Procédé pour nettoyer la peau, **caractérisé en ce qu'**il consiste à appliquer sur la peau une émulsion nettoyante selon l'une quelconque des revendications 1 à 10.

## Claims

1. Oil-in-water cleansing emulsion having the appearance of a milk, **characterized in that** it has a viscosity equal to or less than 1 Pa s and **in that** it comprises a self-emulsifiable composition based on fatty alcohols as emulsifying agent and an acrylic copolymer as gelling agent, which acrylic copolymer is a crosslinked copolymer of a C₃-C₆ monoethylenic acid or of the anhydride thereof with an acrylic ester.

2. Cleansing emulsion according to Claim 1, **characterized in that** the monoethylenic acid has the formula: 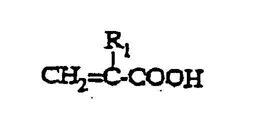 where R₁ represents a group chosen from hydrogen or the halogen, hydroxyl, lactone, lactam, cyanogen, alkyl, aryl, aralkyl, alkylaryl and cycloaliphatic radicals.

3. Cleansing emulsion according to Claim 1 or 2, **characterized in that** the acrylic ester has the formula: 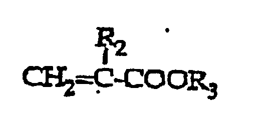 where R₂ represents a group chosen from hydrogen or the methyl or ethyl radicals, and R₃ represents a group chosen from C₈-C₃₀ alkyl, C₈-C₃₀ oxyalkylene and C₈-C₃₀ carbonyloxyalkylene radicals.

4. Cleansing emulsion according to any one of Claims 1 to 3, **characterized in that**, in the copolymer, the proportion of monoethylenic acid is from 50 to 99% by weight and the proportion of acrylic ester is from 50 to 1% by weight.

5. Cleansing emulsion according to any one of Claims 1 to 4, **characterized in that** the copolymer is a copolymer of C₁₀-C₃₀ alkyl acrylate and of acrylic or methacrylic acid, crosslinked with an allyl ether of sucrose or of pentaerythritol.

6. Cleansing emulsion according to any one of the preceding claims, **characterized in that** the self-emulsifiable composition comprises from 60 to 90% by weight of at least one fatty alcohol having from 12 to 22 carbon atoms, from 10 to 40% by weight of an alkylpolyoside whose alkyl chain has from 12 to 22 carbon atoms, and from 0 to 5% by weight of polyoside.

7. Cleansing emulsion according to any one of the preceding claims, **characterized in that** it comprises from 0.5 to 5% by weight of self-emulsifiable composition relative to the total weight of the emulsion.

8. Cleansing emulsion according to any one of the preceding claims, **characterized in that** it comprises from 0.02 to 0.5% by weight of copolymer relative to the total weight of the emulsion.

9. Cleansing emulsion according to any one of the preceding claims, **characterized in that** it comprises from 2 to 30% by weight of oil.

10. Cleansing emulsion according to any one of the preceding claims, **characterized in that** it additionally comprises at least one adjuvant chosen from preserving agents, antioxidants, fragrances, fillers, screening agents, sequestering agents, essential oils, dyes, hydrophilic or lipophilic active agents, and lipid vesicules.

11. Make-up removing milk, **characterized in that** it contains an emulsion according to any one of Claims 1 to 10 and a make-up removing oil.

12. Use of the cleansing emulsion according to any one of Claims 1 to 10 for cleansing the skin.

13. Process for cleansing the skin, **characterized in that** it consists in applying to the skin a cleansing emulsion according to any one of Claims 1 to 10.

## Patentansprüche

1. Öl-in-Wasser-Reinigungsemulsion, die das Aussehen einer Milch aufweist,
**dadurch gekennzeichnet, daß**
sie eine Viskosität von 1 Pa·s oder darunter aufweist und sie als Emulgator eine selbstemulgierende Zusammensetzung auf der Basis von Fettalkoholen und als Gelbildner ein Acrylcopolymer enthält, bei dem es sich um ein vernetztes Copolymer einer einfach ethylenisch ungesättigten Säure mit 3 bis 6 Kohlenstoffatomen oder eines Anhydrids davon und eines Acrylesters handelt.

2. Reinigungsemulsion nach Anspruch 1, **dadurch gekennzeichnet, daß** die einfach ethylenisch ungesättigte Säure die Formel 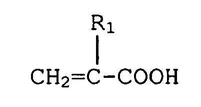 aufweist, worin R₁ einen Substituenten bedeutet, der unter Wasserstoff, Halogen, Hydroxy-, Lacton-, Lactam-, Cyano-, Alkyl-, Aryl-, Aralkyl- und Alkylarylgruppen sowie cycloaliphatischen Gruppen ausgewählt ist.

3. Reinigungsemulsion nach Anspruch 1 oder 2, **dadurch gekennzeichnet, daß** der Acrylester die Formel 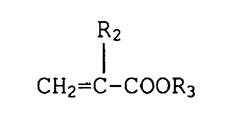 aufweist, worin R₂ einen Substituenten, der unter Wasserstoff oder Methyl oder Ethyl ausgewählt ist, und R₃ eine Gruppe bedeutet, die unter C₈₋₃₀-Alkyl, C₈₋₃₀-Oxyalkylen und C₈₋₃₀-Carbonyloxyalkylen ausgewählt ist.

4. Reinigungsemulsion nach einem der Ansprüche 1 bis 3, **dadurch gekennzeichnet, daß** in dem Copolymer der Anteil der einfach ethylenisch ungesättigten Säure 50 bis 99 Gew.-% und der Anteil des Acrylesters 50 bis 1 Gew.-% beträgt.

5. Reinigungsemulsion nach einem der Ansprüche 1 bis 4, **dadurch gekennzeichnet, daß** das Copolymer ein Copolymer von C₁₀₋₃₀-Alkylacrylat und Acrylsäure oder Methacrylsäure ist, das mit einem Allylether von Saccharose oder Pentaerythrit vernetzt ist.

6. Reinigungsemulsion nach einem der vorhergehenden Ansprüche, **dadurch gekennzeichnet, daß** die selbstemulgierende Zusammensetzung 60 bis 90 Gew.-% mindestens eines Fettalkohols mit 12 bis 22 Kohlenstoffatomen, 10 bis 40 Gew.-% eines Alkylpolysaccharids, dessen Alkylkette 12 bis 22 Kohlenstoffatome aufweist, und 0 bis 5 Gew.-% Polysaccharid enthält.

7. Reinigungsemulsion nach einem der vorhergehenden Ansprüche, **dadurch gekennzeichnet, daß** sie 0,5 bis 5 Gew.-% der selbstemulgierenden Zusammensetzung, bezogen auf das Gesamtgewicht der Emulsion, enthält.

8. Reinigungsemulsion nach einem der vorhergehenden Ansprüche, **dadurch gekennzeichnet, daß** sie 0,02 bis 0,5 Gew.-% Copolymer, bezogen auf das Gesamtgewicht der Emulsion, enthält.

9. Reinigungsemulsion nach einem der vorhergehenden Ansprüche, **dadurch gekennzeichnet, daß** sie 2 bis 30 Gew.-% Öl enthält.

10. Reinigungsemulsion nach einem der vorhergehenden Ansprüche, **dadurch gekennzeichnet, daß** sie ferner mindestens einen Hilfsstoff enthält, der unter Konservierungsmitteln, Antioxidantien, Parfums, Füllstoffen, Filtern, Maskierungsmitteln, etherischen Ölen, Färbemitteln, hydrophilen oder lipophilen Wirkstoffen und Lipidvesikeln ausgewählt ist.

11. Milch zum Abschminken, **dadurch gekennzeichnet, daß** sie eine Emulsion nach einem der Ansprüche 1 bis 10 und ein Öl zum Abschminken enthält.

12. Verwendung der Reinigungsemulsion nach einem der Ansprüche 1 bis 10 zur Reinigung der Haut.

13. Verfahren zur Reinigung der Haut, **dadurch gekennzeichnet, daß** es darin besteht, eine Reinigungsemulsion nach einem der Ansprüche 1 bis 10 auf die Haut aufzutragen.
